# EUROPEAN PATENT APPLICATION

(11) **EP 3 639 738 A2**
(43) Date of publication of application: **22.04.2020**
(21) Application number: 19461608.2
(22) Date of filing: 22.11.2019
(51) Int. Cl.: A61B 5/00, A61B 5/055

(54) **SYSTEM FOR GIVING FEEDBACK BASED ON MOTION BEFORE OR DURING MEDICAL IMAGING AND CORRESPONDING METHOD**

(71) Applicant: Neuro Device Group S.A., 04-501 Warszawa (PL)
(72) Inventor: Soluch, Pawe, 04-501 Warszawa (PL); Orzechowski, Mateusz, 04-453 Warszawa (PL); Malej, Krzysztof, 04-453 Otwock (PL); Obr bski, Wojciech, 08-110 Siedlce (PL); Rogowski, Pawe, 04-672 Warszawa (PL); Wrotkowski, Krzysztof, 20-075 Lublin (PL)
(74) Representative: Schmidt, Christian

(57) **Abstract**

Disclosed is a system (100, 200) for giving feedback based on motion before or during medical imaging, comprising:
- an optical camera device (110, 210) that is configured to generate image data of at least two images of a subject (408) or of a part of a subject (408) which can be arranged or is arranged at a subject placing location (194) of a medical imaging device (192), and
- a feedback signaling unit (120, 124) that is configured to generate based on movement data obtainable from the image data a feedback signal (Si1, Si2) that is perceptible by the subject (408) and/ or by an operator of a medical imaging device or by MRI technician (192).

## Description

The disclosure relates to a system for giving feedback based on motion before or during medical imaging, for instance MRI (magnetic resonance imaging) or CT (Computer Tomography).

Known MRI machines (also named as MRI scanners or MRI devices) are used to take medical images of various parts of human body or internal organs, e.g. brain. These may be used either for clinical purposes, as well as scientific purposes. Structural images present information on structure of an organ and could help to identify possible abnormalities, e.g. tumors. Furthermore, other modalities of MR imaging allow to perform analysis of other features of the scanned tissue, including fMRI (functional MRI) to analyze activity of the brain or MRS (Magnetic Resonance Spectroscopy) to study metabolic changes within the tissue. Depending on the modality and the purpose, the imaging procedure may take from single minutes to multiple hours, divided into various sequences. During data registration a patient or subject should remain still in order to avoid movement artifacts in the obtained data.

Principles of MRI machine operation rely on the fact, that certain atomic nuclei are able to emit radio frequency energy when placed in an external magnetic field and/or when stimulated appropriately. In clinical and research MRI, hydrogen atoms may be most often used to generate a detectable radio-frequency signal that is received by coils (antennas) in close proximity to the body of the subject being examined. Hydrogen atoms are naturally abundant in people and other biological organisms, particularly in water and fat of a living body. The field strength of the magnetic field that is used in MRI machines is for instance within the range from 0.5 T (Tesla) to 11.7 T, especially within the range from 0.8 T to 7 T. Examples are for instance clinical MRI machines operating with a field strength of 0.8 T, 1.5 T or 3 T.

Thus the MRI machine may comprise strong magnets to generate a strong static magnetic field. Furthermore, oscillating magnetic fields and/or radio frequency (RF) fields may be generated in order to generate the radio frequencies signals that are based on nuclear spins. Radio frequency coils may receive these signals. These properties are important technical challenges for any technologies used in proximity to the MRI machine. Furthermore reduction of artifacts further limits possibilities of performing various activities in the proximity, such as controlling of the subject.

MR images, i.e. 3D (three dimensional) image data, comprise voxels (volume element), that may be formed by gradient coding and excessive computing may be used to associate received RF signals with corresponding voxels. Usage of 2D (two dimensional) or 3D Fourier transformation may be possible for instance to generate image data. Voxel size may be important to data quality and may be e.g. 1 mm (millimeter) by 1 mm by 1 mm or 0.5 mm by 0.5 mm by 0.5 mm.

The gantry or tube of an MRI machine may be a space in which the patient or subject or parts of his/her body are placed during MRT (Magnetic Resonance Tomography). The gantry or the subject placing location may form a ring, tube or hollow cylinder. Other types of subject placing locations may be used as well. This limits access to the patient or subject and possibilities to monitor/control his condition.

Some of the problems mentioned in this application may be also valid for CT machines (also named as CT scanners or CT devices) that use x-ray and that reconstruct digital images from a plurality of images that are taken from different directions. In general, CT imaging may be performed faster than MRT imaging.

For example, the head of the subject has to be placed within a narrow tube of the MRI or the CT scanners. This makes it difficult to take for instance optical images or a video stream of the face of the subject. Furthermore, it is difficult to communicate with the subject during the MR imaging process. Other persons are not allowed to be within the room in which the MRI machine is located, especially during MRT.

One of the main challenges is to make sure that the subject or patient does not move during the medical imaging process. This movement may prevent correct imaging and may lead therefore to unnecessary repetitions of the medical imaging procedure.

It is an object of the invention to provide a system that prevents or mitigates movements of the subject before and/or during medical imaging. The system shall be simple and/or cost efficient and/or provide maximal comfort to the subject or patient. Furthermore, a corresponding method and other corresponding technical devices shall be given.

This object is solved by the system according to claim 1. Further embodiments are given in the dependent claims. Furthermore, the object is solved by the subject matter of the independent claims.

### Summary of the invention:

In one aspect the system may comprise the following features:
- an optical camera device that is configured to generate image data of at least two images or "optical" images of a subject or of a part of a subject which is placed, preferably simultaneously, at a subject placing location of a medical imaging device, and
- a feedback signaling unit that is configured to generate based on movement data obtainable from the image data a feedback signal that is perceptible (perceivable) by the subject and/ or by an operator of a medical imaging device and/or by a technician that is responsible for the medical imaging device.

### Embodiments

In a first aspect the system may comprise the following features:
- an optical camera device that is configured to generate image data of at least two images or "optical" images of a subject or of a part of a subject which is placed, preferably simultaneously, e.g. at the same time or at the same moment, at a subject placing location of a medical imaging device, preferably a non-optically image generating medical imaging device,
- preferably an optional image processing unit that is coupled to the optical camera device and that is configured to process the at least two optical images in order to determine the movement of the subject or the part of the subject before or during medical imaging (medical image generation),
   wherein preferably the optional image processing unit generates at least one output signal depending on the determined movement,
- a feedback signaling unit that is preferably coupled to the image processing unit and that is configured to preferably receive the at least one output and to generate a feedback signal that is perceptible by the subject and/ or by an operator of a medical imaging device and/or by a technician that is responsible for the medical imaging device.

The invention is based on the idea that feedback based on motion should be given to the subject already before and/or during the imaging process. Thus, the subject has the possibility to adapt its movements as required for a fault free medical imaging process. Therefore, a signal feedback method may be used.

The invention is also based on the consideration, that the feedback should be generated as fast as possible and/or with only a small additional effort. Therefore, an optical camera is used that allows fast and simple optical image generation compared to the slower and computational extensive image generation for medical imaging, for instance MRI or CT. The MRI technician or an operator of medical imaging machine who is performing the procedure may also receive the feedback signal and/or may only receive the feedback signal, i.e. the subject may not receive the feedback signal. Therefore, the technician or the operator may be less engaged in controlling the movement of the subject.

The output signal may be an analog output signal or a digital output signal, i.e. comprising data. The output signal or output data may be transmitted by wire, wireless or via an optical fiber from the image processing unit to a signaling unit. Optical fiber has the advantage, that it does not disturb the medical imaging process and that it is not disturbed by such processes.

The amount of motion or movement that is determined may be compared to a threshold. However, there may be other possibilities that do not involve a threshold in order to quantify the movement. Difference pictures (i.e. subtracting corresponding pixel intensity values from each other), implicit markers on the subject (detection of chin, nose or other parts, beauty patch, etc.), additional marker on subject (color markers, stickers) may be used in order to ease motion detection. Furthermore, it is possible to define a reference or desired position and to calculate an actual position in order to determine movement.

The feedback may be given for instance by an optical and/or acoustic signaling device. Vibration or other kinds of signaling may be used as well. A computer game may be used for feedback in which for example an object or a character is adjusting its behavior according to the movement of the subject. The objective of the patient or subject may be to maintain a certain state of the object or the character in the game.

The optical range may be defined as the range of 400 to 700 nm (nanometers). This means that electromagnetic waves having a wavelength within this range may be used. There may be also an illumination device that generates the light for the optical camera, preferably on optical illumination device.

The technical effect that is reached by the invention is that the quality of medical imaging may be raised considerably by using comparably simple technical means.

The feedback signal may change if the orientation and/or location of the subject relative to the medical image device changes by more than a predetermined amount. The feedback signal may indicate that the determined motion is acceptable for the medical imaging. Thus, the subject is motivated to hold still. Alternatively or additionally, the feedback signal may indicate that the determined motion is not acceptable for the medical imaging. Thus, the subject may make more efforts to remain in a still position. Feedback signals that are between these two extrema may be generated as well in order to make the medical imaging procedure as short as possible.

The optical camera device may be configured to operate within a static magnetic field of at least one tesla, at least two tesla or at least three tesla but for instance less than 10 tesla or less than 9 Tesla or less than 8 Tesla. The optical camera may comprise a CCD (Charge Coupled Device) or a CMOS (Complementary "Metal" Oxide Semiconductor) image sensor. Other cameras may be used as well. The optical camera device may be adapted to work properly within or to withstand the MRI magnetic and/or radio frequency fields. Alternatively, the optical camera device may be adapted to work properly or to withstand x-ray if a CT is used for the medical imaging process.

The words "the at least two images" that are mentioned above may refer to any two images of a sequence of images. There may be "optical" images that have been captures between the at least two images within the same series of "optical" images. This means that some images may be skipped and measurement is only performed for instance within a time frame that repeats every second, third, fourth, fifth, sixth image etc. Alternatively, the at least two images may be adjacent with regard to each other within the same sequence of "optical" images, for instance the last two images that were captured or two adjacent images from another position within the sequence of "optical" images. Two images, three images or more than three images may be used to detect the movement of the patient.

The image processing unit may be configured to generate an intermediate score that indicates the movement during a time period that is shorter than the medical imaging sequence. The intermediate score may be shown to the subject and/or to the technician in real time (for instance within a time interval that is less than 2 seconds or less than 1 second), for instance using a color code presented with a single LED (light emitting diode), an LED bar, an LED display or another appropriate output device.

Alternatively or additionally, a final score may be shown or told to the subject after the medical imaging process. The intermediate score and/or the final score may be formed by summing up at least two values or all values that indicate the amount of movement during the time period, e.g. accumulated movement data may be used as a score or as a basis to calculate a score.

Thus, the trend to gamification may be used to motivate the subject to hold its body very still during the medical imaging procedure. The subject may follow his/her play instinct and may be able to get low movement scores. High scores may be used as well using appropriate transformation calculations.

The feedback signaling unit may be integrated into the camera device. This may allow to keep the number of different device units low and/or to be cost efficient. Both units may use the same power unit and/or the same signal transmitting unit and/or signal receiving unit. As the camera is near the subject placing space within the medical imaging device it is easy to transmit feedback signal into the subject placing space if the feedback unit may be comprised within the camera, e.g. within the same housing or case. This embodiment may be used for instance for medical images of the head of a subject, e.g. a person or a patient. If the lens of the camera is seen in a top view the feedback signaling unit may be arranged laterally of the lens of the camera. Thus, it may be visible to the subject if a face of the subject is in the field of view of the cameras, e.g. if images of the face are taken by the camera device.

Alternatively, the feedback signaling unit may be a device that is separate from the camera device. The feedback signaling unit may be integrated within the medical imaging device, e.g. a MRI machine. This embodiment may be used for instance for optical or other feedback to a subject when their head is outside of the subject placing space, for instance if medical images of a limb, of a hip, of a chest or of an abdomen are generated.

The image processing unit may be comprised in or integrated into the optical camera device or in a control unit for the optical camera device, e.g. the image processing unit may be within a radius of 3 meters around the medical imaging device, e.g. mounted or fastened at the medical imaging device. Alternatively, the image processing unit may be integrated with the medical imaging device. The control unit may be operatively coupleable or may be coupled to the camera device. The image processing unit may be configured to operate within a static magnetic field of at least one tesla and/or wherein the image processing unit is configured to emit no or only weak electromagnetic radiation that is able to generate artefacts in medical images of the medical imaging device. This means that the image processing unit may be operated inside of an MRI room. Shielding measures may be applied. Other EMC (Electromagnetic Compatibility) measures are possible as well, for instance electrical filtering of power lines and/or of signal lines.

Shielding may be especially important for a power source. Combinations of switchable electrical power sources and batteries may be used. All power supply lines and/or ground lines may comprise additional filter units in order to fulfill EMC requirements. Integration of the image processing unit into the camera or into the control unit of the camera may reduce the number of modules of the system, i.e. it may save costs for such modules.

Alternatively, the image processing unit may be a device that is separated from the camera device and from a control unit of the camera device. Again the image processing unit may be operatively coupleable to the camera or to the control unit, for instance by wire or by fiber. Separate devices may have their own electrical power unit and/or their own sending unit and/or receiving unit for data or other signals.

The image processing unit may comprise no separate units that would allow operation near to an MRI machine or a CT machine. The image processing unit may be arranged outside of an MRI room in this case. Less shielding measures may be taken, and EMC may be not such a strong issue. Devices that are already in use for other purposes outside the MRI/CT room may also comprise the image processing unit, for instance a computer that is used to monitor the medical imaging procedure or a sending and receiving unit.

The image processing unit may be configured to detect movements that are less than 3 mm, less than or equal to 2 mm or less than or equal to 1 mm. This may allow to detect also small movements that are detrimental for medical imaging processes. The detection threshold may be given by a threshold value. The threshold value may be for instance more than or equal to 0.1 mm, more than or equal to 0.2 mm, more than or equal to 0.3 mm or more than or equal to 0.5 mm in order to prevent the detection of micro movements that may be uncontrollable for the subject or patient and/or reduce the costs of the overall system. Thus, it may be possible to prevent a constant negative feedback.

The image processing unit may be configured to detect movements within a time interval of less than half or equal to half a second or less than or equal to 100 milliseconds between the following events a) and b):
- a) commencement of taking or capturing the last "optical" image that is used for the computations and to the generation of the output, and
b) the generation of the output signal, for instance on an output port.
If medical image data would be used for this purposes, the interval would be significantly longer, as it would be provided only after complex computations of the whole sequence.

Event a) may relate to the beginning of an imaging procedure for the last image, i.e. storing charge in an CCD device or in a CMOS device compared to the start of taking an image for an MRI, e.g. by generating at least one magnetic impulse, followed by calculation of voxels, calculation of image planes, etc.

All image processing may be done on pixel data (picture element). The format of the pixel data may correspond to a standard (Bitmap format BMP or JPEG (Joint Photographic Experts Group) format) or may be a proprietary format, for instance without data compression etc. One embodiment may work on a matrix of values that are coming from digitalized values from an analog camera. In this case there is no need to process standard image formats. In case of frame grabber usage and/or computations on PC (Personal Computer) and/or use of digital output camera - all considerations on Bitmap or JPEG processing may remain valid.

Thus real time or near real time processing is possible enabling fast feedback and consequently a resulting closed loop with small time constants. The quality of the medical imaging process may be significantly improved by this real time feedback method.

The image processing unit may comprise a masking unit or a masking functionality that is configured to identify and/or mask at least one area or region or other feature of the subject, preferably an area of the eyes and/or of the lips of the subject. The image processing unit may be configured to disregard movements inside the at least one area/region/feature. Thus, movement may be detected only outside of the masked areas/region/feature. For instance unavoidable blinking of the eyes may be disregarded in the analysis as movement that is detrimental for the medical imaging process. The same may apply for instance to the movement of the lips. An open software package may be used for these image processing tasks, for instance IntraFace, see literature 3) that is mentioned below. Alternatively, a proprietary solution or a commercial solution may be used.

The areas or regions or features may be determined automatically, e.g. without involvement of a person, or semi automatically. Alternatively, manual masking may be performed.

The image processing for movement detection may be very simple (e.g. image subtraction). Alternatively more complex algorithms may be used to improve accuracy. These algorithms may include, but are not limited to, at least one of the following:
1) ORB, i.e. oFAST (oriented FAST, e.g. using for instance intensity centroids) and/or rBRIEF (rotated BRIEF, e.g. using for instance a rotation matrix), see literature 1),
2) FAST (Features from Accelerated Segment Test, high speed corner detection), e.g. using a circle around a potential corner and classifying the intensities of at least some of the pixels along the circle, for instance into three categories. Building a decision tree that can correctly classify all corners seen in the training set during a training phase.
3) BRIEF (Binary Robust Independent Elementary Features): Smoothing the image patches first and then using a hard comparison test that gives only 0 or 1 for instance. Thus, it is possible to use the Hamming distance for a next comparison. Short descriptors (less than 1000 bits, for instance 128, 256 or 512) may be built by comparing the intensities of pairs of points.
4) SIFT (Scale Invariant key points) which uses local gradient histograms,
5) SURF (Speeded Up Robust Features) which improves the calculation speed of SIFT by using a Hessian matrix-based measure for the detector. SURF may be also improved by using GPU (Graphic Processor Unit) optimizations.
7) image flow detection according to Lucas and Kanade, see literature 2) that is mentioned below.

The system may comprise a triggering functionality, wherein the trigger(s) may be configured to change the operation of MRI machine in response to detected movement.

A first (this should be regarded only as a name and not as numbering) trigger signal may be issued by the image processing unit for an medical imaging system, e.g. an MRI system, to repeat only a part of a current sequence, for instance the past k-space line in case of an MRI machine, if movement of the subject exceeded or exceeds a limit and the subject or patient got back to his/her previous position, e.g. the position at which the movement has been started. Only a part of the current sequence may be deleted or marked as containing images of lower quality. Alternatively or additionally to the first trigger signal, a second (this should be regarded only as a name and not as numbering) trigger signal may be issued by the image processing unit for the MRI system if movement exceeded or exceeds the limit and the subject did not return to his/her previous position.

The second trigger signal may trigger the medical system, e.g. an MRI system, to repeat the whole sequence. There may be systems that make the same comparison based on MRI data after finishing the whole imaging sequence. However, a system like the proposed system that is based on "optical" image data, i.e. on image data generated using an optical camera, may give the feedback in real time and may issue trigger signals during the medical imaging sequence, thus speeding up the overall time of the medical imaging procedure in case of movement of the subject, e.g. of a part of the subject (head, limb etc.). The trigger signal(s) may enable an automated or more automated medical imaging process compared to methods that are based on the MR images and/or that are only performed at the end of the medical image sequence.

A further aspect of the invention relates to an image processing unit, preferably an image processing unit according to one of the embodiments mentioned above. The image processing unit may comprise an input unit, an output unit and a processing unit.

The input unit may be configured to receive image data of at least two images from an optical camera device. The processing unit may be configured to process at least two "optical" images in order to determine the movement of a subject or the part of the subject before or during a medical imaging that is preferably non-optical. The processing unit may be configured to generate at least one output signal or output data depending on the determined movement. The output unit may be configured to send the output data or the output signal to a feedback signaling unit. Thus, the same technical effects are also valid for the image processing unit that are mentioned above for the system and its embodiments. The meaning of "at least two images" is explained above in more detail.

In an embodiment the electronic unit for image processing may comprise:
- a processing module,
- an input data port, used to input image data from an MRI compatible camera to the processing module,
- an output port, used to output the control signal to an output device configured to provide information to the subject undergoing an MRI procedure, e.g. optical output device, and
- a program code implementing, when executed on the processing module, the method for camera based gamification and/or feedback of MRI procedures.

A next aspect relates to a method of giving feedback based on motion before or during medical imaging, e.g. medical imaging, comprising:
- generating (taking or capturing) movement data of a subject or of a part of the subject from which at least one medical image is generated during and/or before generating the at least one medical image,
- giving or sending/transmitting to the subject a feedback signal that depends on the amount of the determined movement.

Thus, the same technical effects may apply to the method that apply to the system and its embodiments. Vice versa, the method steps may be also valid for the system and/or image the processing unit mentioned above.

There may be the situation in that first feedback is provided to the patient and/or to a technician and then, when the subject is sufficiently still, obtaining medical images is started.

A great part of the advantages of the method may arise if the method is performed before and/or during medical imaging. The method may also be applied in a non-medical imaging context or in a context that does not involve image generation at all, i.e. excluding or disregarding the "optical" images of the camera.

The movement data may be generated using an optical camera, using a light barrier, using a motion detection sensor, for instance Infrared or ultrasonic, or using an imaging device that is not based on optical imaging generation. It may be possible to use images that are generated using an MRI machine.

The method may comprise:
- determining movement of the subject or of the part of the subject based on at least two images that are generated optically. The technical effects of imaging processing of images that are generated optically are also valid for the method.

The method may comprise:
- presenting or sending/transmitting a feedback signal using a computer game wherein preferably an object or a character adjusts its movement and/or its behavior according to the movement of the subject. Thus, the feedback signal may be delivered in a way that is maximal adapted to the abilities of the user, e.g. the subject or an MRI technician.

The method may further comprise:
- generating non-optically at least one medical image of the inside of a subject. The non-optical image generation may be more complex than the optical image generation using a camera.

In an embodiment the method for camera based feedback and/or gamification of MRI procedures may comprise:
- movement recognition and tracking, e.g. movement of the head or other part of the body undergoing MRI procedure, preferably based on image data captured by an optical camera,
- classification of a recorded movement level or type to two or more classes,
- issuing a control signal for the output device based on the classified movement class,
- a method, wherein a patient/subject is instructed to control (e.g. to minimize) his/her movement to achieve predefined information or signaling on the feedback signaling unit (e.g. an output device) constantly over a defined time of the procedure, e.g. of the medical imaging procedure.

The medical image may be an MR image of an MRI machine. However, the medical image may also be a CT image or another type of medical image, showing for instance images of inner organs (brain, heart, lung, etc.) or of bones. Although the generation of the medical image may be comparably complicated, the monitoring of the movement is based on optical image generation and is therefore simple and/or fast. The generation of medical images may take a longer time in MR images compared to CT image, i.e. the invention may have greater impact on MR imaging procedures compared to CT imaging procedures.

At least two or at least three movement levels may be defined. The determined movement may be classified according to the specified movement thresholds. Different feedback signals may be generated depending on the different classifications. More, than two movement levels may make it easier for the subject to identify and control his/her movements for a longer time interval in the range of for instance 1 minute to 60 minutes or in the range of 1 minute to 10 minutes.

The subject may be instructed to control and/or to minimize his/her movement to achieve predefined feedback, preferably on a feedback signaling unit and/or preferably constantly over a defined time of a medical imaging procedure. The subject may be a human patient, for instance an adult or a child.

The method may further comprise:
- matching at least one keypoint (marker) within at least two optical images and determining a value that represents the movement of at least one keypoint (marker),
- comparing the value with at least one threshold value,
- giving to the subject a feedback signal that indicates that the value is above the at least one threshold value and/or feeding back to the subject a signal that indicates that the value is below and/or equal to the at least one threshold value.

The keypoint may be a corner or other feature of the subject which is visible using an optical camera. The feedback step may further comprise: generating a signal, transmitting the signal (by wire, optical or wireless), receiving the signal in a signaling unit, for instance in an output device, e.g. an optical output device, and controlling output elements of the output device according to the signal. The at least one generated signal may depend on the result of the comparison. A further step of the method may comprise the placing of the subject or of a part of the subject in a field of view (subject placement location) of a medical imaging device that is able to generate medical images or image sequences (video).

The keypoint may be a prominent feature of the subject, e.g. the keypoint should easily be detectable by image processing. Alternatively, optical markers may be attached to the subject, for instance using stickers, tape or band-aid (may be a registered trade mark). The image processing may associate a digital descriptor to at least one keypoint or respective descriptors to all keypoints that are determined in the image data. Movement recognition and tracking may be performed using the keypoint(s) and/or their descriptors.

A computer program product may comprise computer readable program code with instructions which, when loaded and/or executed on a processor, cause the processor to carry out at least one of, an arbitrarily selected plurality of the method steps according to the methods mentioned above. The usage of a computer program product enables automatic movement detection and feedback.

Alternatively it is possible to use a dedicated hardware that comprises no processor that performs instruction of a program. The dedicated hardware may comprise an ASIC (Applicant specific Integrated Circuit), an FPGA (Field Programmable Array), PLD (Programmable Logic Device), etc. realizing for instance a finite state machine.

A last aspect relates to a system, comprising:
- an optical camera device that is configured to generate image data of at least two optical images of a subject or of a part of a subject which can be arranged at a subject placing location of a medical imaging device, and
at least one trigger unit of:
- a trigger unit (first) that is configured to generate based on the image data a first trigger signal for an medical imaging system, e.g. an MRI system, to repeat only a part of a current image sequence, e.g. the past k-space line in an MRI system, if movement of the subject exceeded a limit and the patient got back to his/her position, and/or
- a trigger unit (second) that is configured to generate a second trigger signal based on the image data for the medical imaging system, e.g. MRI system, if movement exceeded the limit and the subject did not return to his/her previous position. The second trigger signal may trigger the MRI system to repeat the whole medical image sequence.

The system that includes at least one trigger unit may also comprise features of the systems and or methods which are mentioned above, for instance a feedback signaling unit to the subject. However, even without such a feedback signaling unit it is possible to improve the medical imaging process because a sequence may be restarted automatically and/or manually if it is clear that the determined movement of the subject would lead to faulty medical images.

The making and using of the presently preferred embodiments are discussed in detail below. It should be appreciated, however, that the present disclosure provides many applicable concepts that can be embodied in a wide variety of specific contexts. The specific embodiments discussed are merely illustrative of specific ways to make and use the disclosed concepts, and do not limit the scope of the claims.

Moreover, same reference numerals refer to same technical features if not stated otherwise. As far as "may" is used in this application it means the possibility of doing so as well as the actual technical implementation. The present concepts of the present disclosure will be described with respect to preferred embodiments below in a more specific context namely a system for feedback to a subject during magnetic resonance imaging (MRI). The disclosed concepts may also be applied, however, to other situations and/or arrangements as well, for instance for feedback with a subject during other kinds of imaging, especial imaging in a medical context, e.g. CT.

The foregoing has outlined rather broadly the features and technical advantages of embodiments of the present disclosure. Additional features and advantages of embodiments of the present disclosure will be described hereinafter. These features may be the subject-matter of dependent claims. It should be appreciated by those skilled in the art that the conception and specific embodiments disclosed may be readily utilized as a basis for modifying or designing other structures or processes for realizing concepts which have the same or similar purposes as the concepts specifically discussed herein. It should also be recognized by those skilled in the art that equivalent constructions do not depart from the spirit and scope of the disclosure, such as defined in the appended claims.

For a more complete understanding of the presently disclosed concepts and the advantages thereof, reference is now made to the following description in conjunction with the accompanying drawings. The drawings are not drawn to scale. In the drawings the following is shown in:
- Figure 1: an overview over an MRI camera system,
- Figure 2: the general configuration of the video part and of the control part of the MRI camera system,
- Figure 3: a frame head used for carrying parts of the system including a multicolor optical signal device,
- Figure 4: a frame of a holder device,
- Figures 5A and 5B: a method for movement detection, and
- Figure 6: a calculation device.

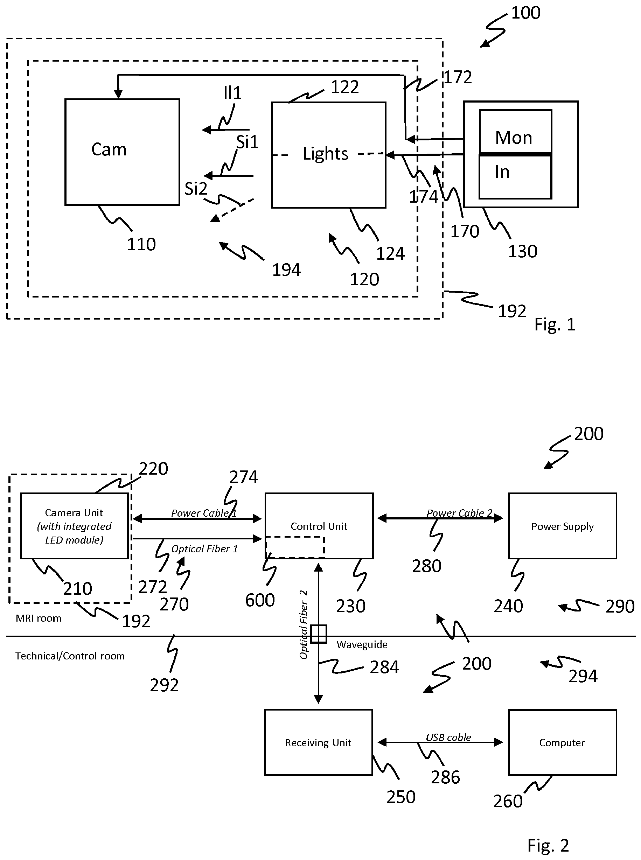

Figure 1 illustrates an overview over an MRI (Magnetic Resonance Imaging) camera system 100. System 100 comprises:
- a camera device 110, for instance for generating an analog signal, for instance PAL (Phase Alternating Line) or NTSC (National Television Systems Committee, US) or SECAM (SEqentielle Couleur A Memoire, FR, RU) or a digital signal. It is possible to generate monochrome or color video signals with camera device 110.
- an optical output device 120, and
- a control unit 130 that may comprise a display or a monitor Mon and an input device In, for instance as part of a touch screen.

Camera device 110, Cam and optical output device 120 may be separate devices. However, in the preferred embodiment camera device 110, Cam and optical output device 120 are arranged within the same housing and it may be said that the optical output device 120 is integrated within the camera device 110.

Camera device 110, Cam and optical output device 120 may be arranged within the interior space 194 of a MRI machine 192 (scanner), i.e. within the inner tube or gantry that is surrounded by big coils that generate a high magnetic field during image acquisition using magnetic resonance tomography (MRT). However, camera device 110 may generate images/pictures or video data using optical sensors, for instance CCD (Charges Coupled Device) or CMOS (Complementary "Metal" Oxide Semiconductor) sensors arranged in a matrix, i.e. in lines and columns. Camera device 110, Cam and optical output device 120 may have to fulfill requirements with regard to MRI shielding and compliance, i.e. they should work properly within high magnetic fields and they should not disturb the MRT.

Camera device 110, Cam and optical output device 120 may be removable or removably placed within MRI machine 192. A connection segment 170 may connect control unit 130 to camera device 110 and to optical output device 120. Connection segment 170 may comprise flexible cables that form a first connection 172 between control unit 130 and camera device 110 and a second connection 174 between control unit 130 and optical output device 124. However, both connections 172 and 174 may end at camera device 110 if optical output device 120 is integrated within camera device 110.

Optical output device 120 may comprise an illumination unit 122 and a signaling unit 124. Illumination unit 122 may comprise light sources, for instance for white light, or other radiation sources (for instance IR (Infrared) radiation) that radiate electromagnetic radiation 111 into the field of view of the camera device 110 enabling recording of optical images thereby.

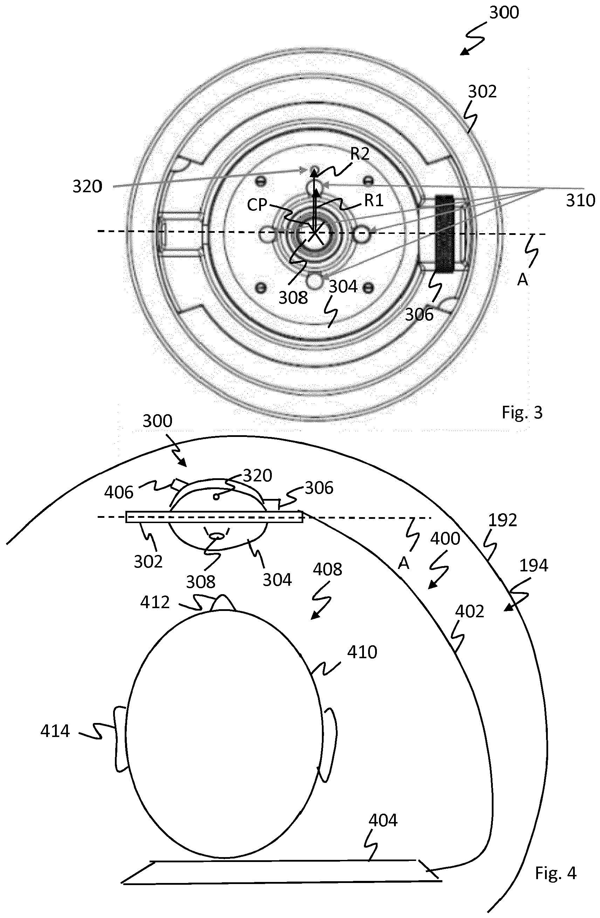

Signaling unit 124 may comprise a light source that generates light that is used for signaling purposes. The light generated by signaling unit 124 may also be directed mainly to the field of view (FOV) of the camera of camera device 110, see signaling light Si1. This may be the case, for instance if the face of the patient is within the focus of the camera of camera device 110. Alternatively, light generated by signaling unit 124 may be directed mainly to a region that is not within the focus of the camera, see signaling light Si2, for instance if the chest of the patient or subject is within the focus but the signaling light has to be seen by the eyes of the patient. One example for the arrangement of camera device 110, illumination unit 122 and a signaling unit 124 is shown in Figure 3 that is described below.

Control unit 130 may comprise an output unit Mon, for instance a screen, display, a monitor or a touchscreen, for showing the video stream that is generated by camera device 110 to an operator or user. Furthermore, control unit 130 may comprise an input device "In" that is used to enter control instructions and/or control data, for instance switching on/off illumination light, switching on/off signaling light, for instance using different colors, selecting video mode of camera (PAL, SECAM, NTSC), etc. Input device In may also be a touchscreen or other input device, for instance a keyboard.

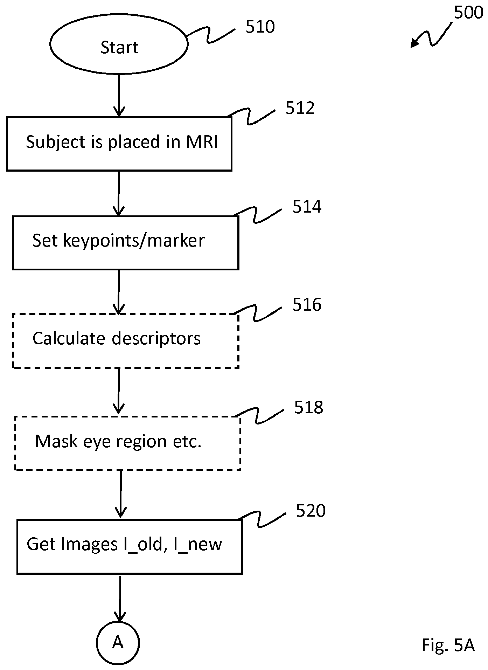

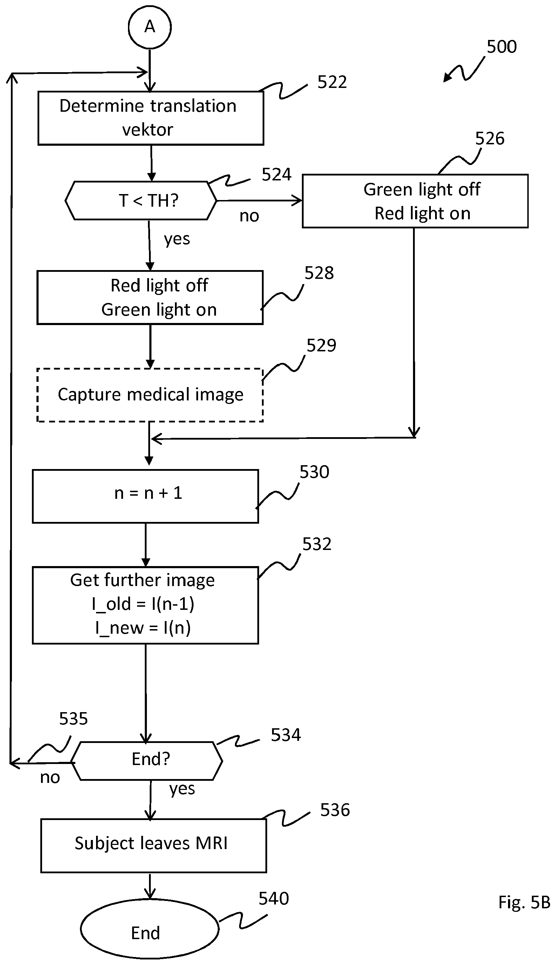

System 100 may be a system which comprises a recording camera device 110 designed for diagnostics and testing in MRI scanners 192. The use of the camera device 110 may increase the safety of the test subjects or of patients and the effectiveness of MR (magnetic resonance) tests or of MRT. It may allow one to see the patient or subject during MRI and fMRI (functional MRI) tests/imaging and may provide feedback on their activity. Alternatively and/or additionally, a special kind of feedback may be movement feedback. This is described in more detail below with regard to the method that is shown in Figure 5.

The system may include or comprise a camera device 110, an output device Mon (monitor) and a lighting system 120 mounted for instance on and/or in the camera device 110.

The camera of the camera device 110 may allow watching the face or other parts of the patient's or subject's body during the MRI scanning procedure. The camera device 110 may provide feedback about the activity of the patient. The camera device 110 may also allow the patient to be observed by the investigator or, in the case of procedures done with children, by the parents. Alternatively and/or additionally, the camera device 110 may be used to generate image data that is used for movement detection or determination of the subject or of the part of the subject that is in the interior space 194 (gantry). The determined level of movement may be the basis for the movement feedback to the subject 408. This is also described in more detail below with regard to the method that is shown in Figure 5.

An output device Mon (monitor), for instance a touch screen, may be used for viewing the image and setting the lighting parameters. The output device Mon and/or the control device 130 may be mounted on the MRI scanner's 192 housing. The touch screen or another input device "In" may allow the examiner or investigator to adjust some of or all settings of the camera that may be placed inside of the gantry, i.e. within the tube, without leaving the MRI scanning room, making their work easier and more convenient.

Lights may be mounted on and/or within the camera housing, see Figure 3, reference numeral 304. The lights may be operated with the input device "In", for instance with a touch screen. The lights may allow additional lighting of the face of the patient, for instance using white light. Infrared lighting may be useful in case of studies requiring darkness. Multicolored light signals may enable communication and may significantly simplify conducting a variety of MRI or fMRI studies or may be used for other purposes.

An image processing unit IPU that is used for movement detection may be comprised within camera device 100 or within control unit 230.

Figure 2 illustrates the general configuration of the video part and of the control part of a MRI camera system 200 that may comprise more devices compared to system 100. System 200 may comprise:
- a camera device 210 that may correspond to camera device 110 and that may comprise the same features that are described above, and/or
- an optical output device 220 that may correspond to optical output device 120 and that may comprise the same features that are described above, and/or
- a control unit 230 (display, monitor, touch screen) that may correspond to control unit 130 and that may have the same features that are described above, and/or
- a power supply device 240 that may generate the electrical power for control unit 230 and/or for camera device 210 and/or for optical output unit 210, and/or
- an optional sending and receiving unit 250, and/or
- an optional computing device 260, for instance a computer, preferably a work station computer.

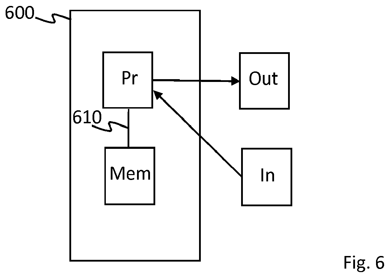

An image processing unit IPU, see for instance Figure 6, that is used for movement detection may be comprised within camera device 200, within control unit 230, within sending and receiving unit 250 or within computing device 260. Alternatively, image processing unit IPU that is used for movement detection may be a separate unit that is used in addition to the other units and/or devices of system 200.

Sending and receiving unit 250 may be a separate unit or may be part of computing device 260, i.e. using the same internal power supply unit, being arranged within the same housing etc.

There may be the following connections within system 200:
- a connection segment 270 between control unit 230 and optical output device 220 / camera device 210. Connection segment 270 may correspond to connection segment 170 (see features mentioned above) and may comprise an optical connection 272 (may correspond to 172) and a power line connection 274 (may correspond to 174), for instance via an electrical cable or line, and/or
- a power line connection 280 that delivers electrical current and electrical voltage from power supply 240 to control unit 230, for instance an electrical conductive cable or line, and/or
- an optional optical connection 284 between control unit 230 and sending and receiving unit 250, and/or
- an optional connection 286 or a wireless connection between sending and receiving unit 250 and computer 260, for instance a USB (Universal Serial Bus) connection.

A splitting unit 600 may be comprised within control unit 230. The splitting unit 600 may comprise at least one or at least two optical splitters, for instance 50% / 50% splitter units each having four ports. The splitting unit 600 may allow the forwarding of data within system 200. The splitting unit 600 is used in the embodiment that is shown in Figure 2. However, there may be embodiments that do not use a splitting unit 600 but use other measures for signal communication. Especially, the method that is shown in Figures 5A and 5B may be performed using a different system than that shown in Figure 2 or in the other Figures, e.g. a system that does not contain a splitting unit 600.

An MRI machine room 290 may comprise: MRI machine 192, optical output unit 210 (arranged within an interior space 194 that is surrounded by MRI machine 192), camera device 220 (arranged within interior space 194 that is surrounded by MRI machine 192) and power supply device 240. Optical output unit 220, camera device 210 and power supply device 240 may be MRI shielded/protected in order to guarantee proper operation during MRT imaging process and in order to prevent artefacts within the MRT image due to the operation of these devices. Alternatively, power supply device 240 may be located outside MRI machine room 290. Furthermore, all power lines may comprise additional electrical filtering.

A wall 292 may separate MRI machine room 290 from a control room 294. Wall 292 may have special shielding, for instance magnetic shielding or EMC (Electro Magnetic Compatibility) shielding. Alternatively or additionally, wall 292 may have an appropriate thickness and/or material, for instance armored concrete. Control room 294 comprises sending and receiving unit 250 and computing device 260 and/or optionally power supply device 240. This also means that sending and receiving unit 250 and computing device 260 and/or power supply device 240 in control room 294 do not have to fulfill special requirements with regard to MRI shielding/protection.

Thus, a communication between a touch screen unit or control unit 230, a receiver unit (sending and receiving unit 250) and a camera device 210 is described. Control unit 230 and sending and receiving unit 250 may allow controlling some or all camera setting options of the camera within camera device 210 and receiving of video signals. All control signals and/or video signals may pass through touch screen unit, i.e. through control unit 230. Thus, control and monitoring of image/video data may be possible from control unit 230 and from computing device 260. Alternatively, it may only be possible to enter control data using control unit 230 or computing device 260. Furthermore, it is possible to operate the light sources of optical output device 220 using control unit 230 and/or computing device 260, for instance for communication with the person or patient who is examined within MRI machine room 290, i.e. sending signals to this person.

Figure 3 illustrates a frame head 300 for carrying parts of system 100 or 200 including for instance only one multicolor optical signal device (for instance RGB (Red Green Blue) LED (Light Emitting Diode), at least one multicolor optical signal device, only one signal device (for instance red LED, green LED or blue LED) or a plurality of signal devices (for instance several LEDs of a different or of the same color).

Frame head 300 may comprise:
- an outer ring 302,
- a housing 304,
- an optional operating element 306,
- a camera 308 of camera device 110, 210,
- at least one illuminating device 310, i.e. one, two, three, four or more than four, and
- only one or at least one signaling device 320, i.e. one, two, three, four or more than four.

Outer ring 302 may have a circular or elliptical shape. Outer ring 302 may be used to mount and hold housing 304 relative to an arm of a frame that comprises frame head 300, see also Figure 4.

Housing 304 may comprise camera device 110, 210 and optical output unit 120, 220. Housing 304 may have a disc shape or a disc like shape. There may be only a narrow gap between outer ring 302 and housing 304 enabling a good protection of the housing, especially of the breakable camera 308 against mechanical impact.

Operating element 306 may be mounted to housing 304, i.e. if operating element 306 is rotated or turned, housing 304 pivots or rotates around an axis A with regard to outer ring 302. Housing 304 may be tilted relative to outer ring 302, see Figure 4. This movement may allow proper positioning of camera 308 and/or of illuminating lights and/or of signaling light(s). Operating element 306 may be an engrailed disc in order to ease operation thereof.

Camera 308 may be part of camera device 110, 210. Camera 308 may allow use of several interchangeable photographic objectives or lenses of different angels of view and/or different focal lengths. Alternatively only one lens may be used. An aperture of camera 308 may be located on a central axis of housing 304 that may be arranged coaxially with outer ring 302 if both parts are within the same plane.

In the example, there are four illuminating devices 310 that may be part of illuminating unit 122 or of a corresponding illuminating unit of optical output device 220. Preferably, optoelectronic devices are used as illuminating devices 310, for instance LEDs. It is possible to use LEDs that radiate white light and/or LEDs that emit IR (infrared) radiation. Alternatively, other types of illuminating devices may be used, for instance lamps with or without a filament.

Four illuminating LED modules 310, for instance arranged crosswise, may be used in the example that is shown in Figure 3. Each illuminating LED module may comprise or contain one white LED and one IR LED. Alternatively, only one of these LEDs may be used in each module, for instance only white LEDs, only IR LEDs or some module(s) only with white LED(s) and other module(s) only with IR LED(s).

In the example shown in Figure 3 only one signaling device 320 is used. Signaling device 320 may be part of signaling unit 124 or of a corresponding signaling unit of optical output device 220. Preferably, optoelectronic devices are used as illuminating devices 320, for instance LEDs. It is possible to use LEDs that radiate white light, colored light of a single wavelength or narrow wavelength band (less than for instance 50 nm), or that radiate multicolored light (for instance two, three or more than three small narrow wavelength band, each less than for instance 50 nm). RGB LEDs or multicolor LEDs may be used to radiate multicolor light, i.e. a mix of several colors. Alternatively, other types of signaling devices may be used, for instance lamps with or without a filament or rotating disks carrying areas of different colors. Only one color area may be visible through an aperture if the disc is in its corresponding angular position. The rotating disk may be illuminated directly or indirectly.

The RGB (Red Green Blue) LEDs may be driven by a PWM (Pulse Width Modulated) controlled current source, preferably by a voltage controlled current source. Alternatively, it is possible to use digital analog converters (DAC) of a microprocessor or separate DACs to control the current source. Other examples may comprise more than one RGB LED module or single LEDs of different colors.

In the example shown in Figure 3, there is the following arrangement of elements:
- the aperture of camera 308 of camera device 110, 210 is arranged centrally within housing 304 wherein a center point CP is arranged in the center of the aperture/lens of the camera 308 (optical axis),
- illuminating devices 310 are arranged at a radius R1 from center point CP and neighboring illuminating devices 310 may have the same distance especially the same angular distance. This may also be valid if less than four or more than four illumination devices are used.
- signaling device 320 is arranged at a radius R2 from center point CP. Radius R2 may be greater than radius R1, for instance by at least 10 percent of radius R1. Furthermore, the radiation characteristic of signaling device 320 that is comprised in signaling unit 124 or a corresponding unit of optical output device 220 may be adapted to radiate away from illuminating devices 310 in order to ease recognition of the signaling by the subject 408 or patient.

Housing 304 may comprise further parts, for instance screws for holding two or more parts of housing 304 together, or parts that are placed on the rear side that is not visible in Figure 3.

Figure 4 illustrates a frame 400 that forms a holder device for housing 304. Frame 400 may comprise:
- frame head 300,
- an arm 402, and
- a foot plate 404 that forms a base.

A connection port 406 may be arranged onto housing 304. Connection port 406 may be used to connect optical connection 172 or 272 to housing 304. Furthermore, connection port 406 may be used to connect power cable 174 or 274 to housing 304. Optical connection 172, 272 and power connection 174, 274 may be combined into one physical cable. Connection port 406 may then comprise an optical connection and electrical connection. Power cable 174, 274 and optical connection 172, 272 may be connected to housing 304 in various ways, for instance using arm 402 or parts of arm 402 for guiding the cable 174, 274.

An inner tube of MRI machine 192 is also shown in Figure 4. Furthermore, the head 410 of a subject 408, e.g. a person or patient, is shown. The inner tube surrounds interior space 194. Head 410 is placed within interior space 194. Figure 4 shows nose 412 and ears 414 of subject 408. Arm 402 of frame 400 may be curved and may be adapted to the shape of the head 410 and/or to the shape of inner tube of MRI machine 192. Head 410 may be placed on foot plate 404 of frame 400 thereby also fixing frame 400 to a bed on which the patient lies. There may be no further mounting means for mounting frame 400 to the bed. Alternatively, further mounting/fixation means may be used, for instance clamping devices. Additional components of frame 400 should not or may not be ferromagnetic nor conductive.

Signaling device 320 may be located nearer to the eyes of subject 408 or patient than illuminating devices 310 in order to ease recognition of the signaling. The nose 412 of the patient is nearer to the head 300 of frame 400 than the back of head 410 of the patient, i.e. the back of head 410 rests on foot plate 404. Foot plate 410 of frame 400 may be upholstered. The distance between head 300 of frame 400 and foot plate 404 may be in the range of 30 cm (centimeters) to 50 cm.

Figures 5A and 5B illustrate a method 500 for movement detection. As is visible from Figure 5A, method 500 starts in a step 510. Various preparation steps may be performed, for instance a variable n may be set to the value one or zero and may be used as a loop counter in the following. Each value of n may correspond to one image that is captured by camera device 110, 210. The steps of method 500 may be performed each after the other if not stated otherwise.

In a method step 512 subject 408 is placed in MRI machine 192. If images of only a part of the body of subject 408 should be taken then this part is placed in the interior space 194 of MRI machine 192. Subject 408 may be instructed to solve a task that involves the signals that are send by signaling unit, e.g. optical output device 220. The task may be to avoid red lights. A more specific task may be to get a movement score that is as less as possible during the whole medical imaging procedure. Alternatively and or additionally, stickers or markers may be attached to subject 408.

In a method step 514 the first image or several images are captured by camera device 110, 210. Image processing is performed by the image processing unit (IPU). Keypoints (markers) may be automatically determined.

In a method step 516 the IPU may calculate digital descriptors for each keypoint. The digital descriptors may enable to differentiate between different descriptors and may be used as a basis for matching the descriptors in a series of images, i.e. a first descriptor in the first image to the descriptor having the same values in the second image, optionally a second descriptor in the first image may be matched to a second descriptor in the second image, and so on. Based on these descriptors movement detection/matching or determination may be possible as is described in detail below. Method step 516 may be optional, for instance if the difference of two adjacent images in the sequence is calculated by subtraction or if other methods are used for movement recognition.

In an optional step 518, some of the descriptors, e.g. at least two descriptors, may be associated with masked areas or regions, for instance in order to mask the eyes and/or the lips of subject 408 if the camera 110, 210 captures images of the face of subject 408 during for instance MRI of head 410. Open source, proprietary or commercial software packages may be used for this purpose. The masking may be done automatically, semi-automatically (e.g. an automatic proposal may be generated and manual correction or manual adaption of the area(s) may be performed) or only manually. The masking may make sure, that movement of the eyes and/or of the lids and/or of the lips and/or of eye brows is not recognized as movement that is detrimental for the medical imaging process.

Figure 5B shows the main phase of method 500. In a method step 520 at least two images have been captured using camera device 110, 210. A preparation phase may include method steps 510 to 520. After the preparation phase, the medical imaging procedure may be started. This may be indicated also to subject 408, for instance by switching on the green light of a multicolor LED or switching on a green LED. Alternatively other colors or other ways of communicating the start of the medical imaging procedure may be chosen.

In a method step 522 the IPU may determine for instance a translation vector between the last two images. However, the last three images or a longer series of the last images may be used for movement recognition, for instance based on translation vectors. Masked areas, see for instance optional method step 518, may not be considered in method step 522 in order to consider only relevant movements which may distort the medical imaging process, e.g. rotation of the head but not movement of the eyes.

In a method step 524 it is tested whether the recognized movement or translation T is less than a threshold TH. It is for instance possible to calculate the length of the translation vector between the same descriptors of the same keypoints in two successive images captured by camera device 110, 210. Alternatively, the length of the translation vector may be calculated over a series of more than two images, for instance considering more than three, four or five images. More sophisticated methods may determine the start of a movement and the end of a movement, e.g. a rest point or a point at which the direction of the movement changes as is the case with a forward and back movement. The amount of translation may be determined very exactly if a start point and an end point are available by image processing. The translation vector may be calculated from the start point to the end point. The camera device 110 does not move and may therefore form a fixed reference system for determination of the movement, e.g. there is always the same fixed reference point within each image, for instance the lower left corner, the center of the image, etc.

The threshold TH may be selected appropriately, for instance with regard to the largest movement that is still tolerable for the medical imaging process. An example is for instance 0.3 mm or 0.5 mm. In method step 524 the IPU tests whether the calculated movement value T is less than threshold TH. If the calculated movement value T is greater than threshold TH a method step 526 follows immediately after method step 524. This is the case when the movement of subject 408 was too strong. In this case the green light may be switched off in method step 526. Furthermore, the red light may be switched on in order to signal that the subject should try harder not to move. The method goes directly to method step 530 after method step 526, i.e. method steps 528 and 529 are not performed within the same loop 535 of method steps 522 to 534 if method step 526 is performed. If the movement was too strong, the IPU may generate a trigger signal that is sent to the MRI machine in order to stop capturing the current sequence of medical images. Method 500 may also be ended in this case.

A more sophisticated approach may use several trigger signals from the IPU to the MRI machine depending on a specific criteria. One of these criteria is whether the subjects moves back or whether the part of the subject is moved back to its previous position. If yes, it may only be necessary to cancel or to delete some of the recorded data or medical images of the current sequence. The start and the first part of the sequence may be used later for medical purposes. Alternatively, medical images that are distorted because of too much movement of subject 408 may be marked by some additional data. This data may indicate that the quality of the respective medical image is not good. If the subject does not return to its original or previous position after a stronger movement a different trigger signal may be sent to the MRI that indicates that the whole sequence is distorted and that medical imaging has to be repeated. Other trigger signals may be used as well.

Furthermore, it is possible to sum up all values T for the cases in which T is equal to or greater to TH. The sum may be used as an overall score of movement during capturing the complete sequence of medical images. Alternatively, all values of T may be summed up in the score, i.e. independently of the result of the test in method step 524. Intermediate scores may also be signaled to the subject/patient 408.

If the calculated movement value T is less than threshold TH, a method step 528 may follow immediately after method step 524. This is the case if the subject does not move or moves only slightly. In method step 528 it is made sure that the red light is switched off and that the green light is switched on. An action may only be taken if a change of the color of the light is necessary.

An optional method step 529 may follow after method step 528 if and when method 500 is performed during medical imaging. However, it is also possible to perform method 500 before medical imaging or in another application context. In method step 529 a further medical image may be captured by the MRI machine. The medical imaging may be based on non-optical image capturing,

In another embodiment of method 500 the movement T is classified in more than two classes. It is for instance possible to use a third LED or a third color of light in order to signal a movement that is not such intensive as a movement that results in red light.

Method step 530 is performed after method step 529 and after method step 526. The counter variable n is incremented in method step 530, for instance by value 1.

In the following method step 532 a further image may be captured optically by camera device 110, 210. This new image may be tagged or named as image l(n). The previous image may be tagged or named as image l(n-1) from the last loop in which method step 532 has been performed or from the preparation phase.

A method step 534 follows after method step 532. In method step 5340 it may be tested whether the medical imaging procedure is already done, for instance using a timer or another appropriate signal or event.

If the medical imaging procedure is ongoing, method 500 proceeds with method step 522. Thus, method 500 is in a loop 535 comprising the method steps 522 to 534. During performing this loop 535 the medical imaging process is performed and movement recognition is active.

The loop 535 may be left in method step 534 only then if the medical imaging process is done. In this case, a method step 536 follows immediately after method step 534. In method step 536 the subject may leave the MRI machine 192 or may remove the body part from the MRI machine 192. Subject 408 may leave MRI machine room 290. Subject 408 may be interested in knowing the score that he/she has reached. The score may be told to the subject and a reward may be given. Method 500 may end in a method step 540. Method steps 536 and 540 may form an end phase of method 500.

Figure 6 illustrates a calculation device 600 that may perform the method steps which are shown in Figure 5. Calculation device 600 may be used as IPU (image processing unit). Calculating device 600 may comprise:
- a processor (Pr) configured to execute instructions, especially for performing the disclosed calculations,
- a memory (Mem) that is configured to store the instructions and to store data that is used or generated during the execution of the instructions,
- an optional input device (In), for instance a keyboard or a data receiving unit (e.g. via internet or intranet), that is configured to input data that will be stored in the memory (Mem), especially to enter the sample values z,
- an optional output device (Out), for instance a display device or a data sending unit (e.g. via internet or intranet), that is configured to output data that is generated during the execution of the instructions, and
- a computer program product that performs movement recognition and/or feedback as mentioned above.

There may be a connection/ bus 610 between processor Pr and memory Mem. Further units of calculation unit 600 are not shown but are known to the person skilled in the art, for instance a power supply unit, an optional internet connection, etc. Alternatively, a server solution may be used that uses calculation power and/or memory space available on the internet supplied by other service providers or on an intranet of a company.

### Example communication of video signals and of control signals over optical fiber

Although, a specific embodiment is given in the following there are plenty of ways to realize the invention using other communication systems and/or protocols. To provide a good performance and easy to use setup in MRI (Magnetic Resonance Imaging) environment, sending and receiving unit 250 should or may send and receive control signals to/from control unit 230 (for instance comprising a touch screen) through optical connection 284 (fiber), i.e. passing through an electromagnetic waveguide for light. To avoid using multiple optical fibers, a single optical fiber may be used for either transmitting video signal and control signals. Splitting unit 600 located within control unit 230 (touch screen) may combine signals coming from video output and control signals. Optical signals may be transmitted through transmission channels that operate using for instance transmitters HFBR-1414MZ of Broadcom® and receivers HFBR-2416TZ Broadcom®. However, other devices of Broadcom® or of other companies may also be used. These electronic circuits allow a nominal bandwidth of up to 125 MHz (Megahertz). Video signals may use a bandwidth of up to 60 MHz (Megahertz). This may leave higher frequencies unoccupied and suitable to use them for control signal transmission. In order to simplify design, it was proposed that wide bandwidth radio transmitters or transceivers (for example using frequencies of 80 MHz and higher, ex. ADF7020-1 BCPZ of Analog Devices®, or corresponding devices of other companies) may be used to control transmission channels for control signals and/or for video signals. Frequency shift keying may be used to transmit control signals.

It should be noted that MRI scanners may use radio frequencies for their operation and this may lead to noise during the operation of the system. 1.5 T (Tesla) MRI scanners may use frequencies of about 64 MHz while 3 T MRI scanners may operate with radio frequencies of about 128 MHZ (for instance 127.734 MHz).

To avoid any artifacts, it could be necessary to use frequencies above this value, so clearly over the 125 MHz bandwidth of optical channels. It may be highly recommended to avoid using of any signal near MRI work frequency and signals which multiples are near MRI work frequency.

Radio frequency transmitters, however, may have the advantage of being able to operate at low signal-to-noise ratio and have very high dynamic range. A system that may comprise a radio transmitter and optical channels may work even without matching to transmission line's characteristic, i.e. electrical and/or optical, provided that the system comprises separate receive RX and transmit TX lines of radio transmitter (transceiver) and/or that the radio transceiver is voltage controlled. The transceiver circuits may be voltage controlled by a microprocessor, for instance using TTL (Transistor-Transistor Logic) technology. Current control may be used only for some components of the system, especially for some other components than the transceiver, in order to control current changes more precisely. Minimal dynamics of transmission line should or must be about 80 dB (Decibel). Radio transceivers may allow to couple analog or digital video signal with digital control signals in one fiber without both signals degradation. Alternatively, a multi-fiber connection may be used. However more fibers may complicate the connection between control unit 130, 230 (Touch Screen Unit) and sending and receiving unit 250.

However, another transmission system may be used as well, for instance only based on electrical conductive signal transmission or only based on optical signal transmission.

Spoken with other words, an electronic unit for image processing IPU and a corresponding method for camera based feedback of MRI procedures are disclosed to prevent motion artifacts in medical image data.

The camera 110 and the image processing units IPU may be used to track a movement of the subjects 408 head 410 during the MRI procedure. Based on some predefined constraints system 100, 200 may decide if the movement may introduce some artifacts to the medical image and indicate it to the patient using for instance RGB lights. There could be the following meaning of colors:
- Green light - ok, maintain this position,
- Orange - you are moving slightly,
- Red - heavy movement.
This may be intended to be a kind of gamification for the patient or subject 408 that would help to avoid artifacts during MRI procedures.

The feature may be performed simply using a computer application on computer 260 connected to system 100, 200 or within one of the disclosed electronic units of system 100, 200, for instance in control unit 130, 230. This is explained in more detail in the following and also above.

Artifacts caused by motion of subject 408 during MRI procedure may cause difficulties in proper recording and/or analysis of MR images, that may be done e.g. for medical diagnostic. In many cases the procedure needs to be repeated in order to obtain proper MRI data. This may expand the time and costs required for the medical imaging procedures. To avoid motion artifacts, various solutions might be applied, including MRI compatible positioning devices, such as immobilizing pads, frames and masks. However, this kind of solutions still allow micro movements. Moreover, they often cause discomfort of the subject that may lead to an increased amount of micro movements.

The disclosed solution aims in preventing movement of subject 408 in specific time slots of the procedures using a method for feedback or gamification that is using data from MRI compatible optical camera 110 to identify movement of subject 408 and to inform subject 408 about the level of the movement using preferably an optical communication device. The proposed method may influence cognitive engagement of the patient, helping him/her to remain still and improving the comfort during a MRI procedure through reduced use of physical immobilizing devices.

The obvious approach for movement tracking and adjusting image acquisition process or data analysis process would be to use image data that is produced by the MRI machine in order to perform motion tracking. These methods may help to avoid restricting patient's movement. However, they often require precise and complex additional apparatus, as well as present limited capabilities.

The proposed solution is relatively simple in terms of hardware being used. It is also based on hardware that can be used for multiple purposes. Furthermore, it allows for discomfort-free movement prevention/minimization.

### Description of an embodiment of the invention:

An electronic unit for image processing may comprise:
- a processing module,
- an input data port, used to input image data from MRI compatible camera 110 to the processing module,
- an output port, used to output the control signal to a output device configured to provide information to the subject undergoing MRI procedure, e.g. optical output device 120,
- a program code implementing, when executed on the processing module, the method for camera based gamification of MRI procedures.

A method for camera based feedback or gamification of MRI procedures may comprise:
- movement recognition and tracking, e.g. movement of the head 400 or other part of the body undergoing MRI procedure,
- classification of the recorded movement level or type to two or more classes,
- issuing control signal for the output device based on classified movement class,
- a method, where patient/subject is instructed to control (minimize) his/her movement to achieve predefined information on the output device constantly over defined time of the procedure.

The image processing module IPU may be a dedicated electronic unit 130, 230 working together with components disclosed above. The processing module IPU may comprise an embedded system (e.g. using the same power unit and the same input output devices as the dedicated electronic unit 130, 230) for computer vision, comprising for instance an image signal processor for image data processing, e.g. a graphic processing unit GPU. The processing module IPU may be integrated in common housing together with one of the previously disclosed components, preferably with control unit 130, 230 or with receiving unit 250. Alternatively the processing module IPU may be built into a separate housing connected to control unit 130, 203 via optical fiber or other connection systems or connected to the receiving unit 250 with an electrical connection or with another appropriate connection, e.g. USB (Universal Serial Bus) cable.

Alternatively, the processing module IPU may be a computer, for instance a personal computer 260, connected to receiving unit 250, for instance with an USB cable, with computer program code executing image processing and issuing control commands to the system disclosed above.

The method for camera based feedback and/or gamification of MRI procedures may comprise movement recognition and tracking based on pixel images that are generated from the analog or digital video from the camera 110. The method may use one of commonly known methods for optical flow calculation, e.g.:
- 1) ORB feature detector to detect features of the image (Ethan Rublee, Vincent Rabaud, Kurt Konolige and Gary Bradski, "ORB: an efficient alternative to SIFT or SURF," IEEE International Conference on Computer Vision, 2011), preferably combined with
- 2) Lukas-Kanade method to calculate optical flow (B. D. Lucas and T. Kanade, "An iterative image registration technique with an application to stereo vision.", Proceedings of Imaging Understanding Workshop (1981), pages 121 to 130).

Alternatively, a method of frames subtraction may be used. This means that the corresponding pixel values of two successive pixel images are subtracted from one another to get a difference pixel image which may preferably show the movement directly.

The signal that is coming from the camera may be an analog signal or a digital signal. There may be at least two ways of further processing:
- 1. The analog signal may be transmitted to technical room 294, for instance to receiving unit 250. The analog signal may be transformed to a pixel image by a frame grabber device and may be processed by dedicated image processor or a computer 260 with dedicated image processing software.
- 2. In the second option, the analog signal may be digitalized by a circuit in control unit and digitized image data may be processed by an image processor, integrated with the control unit 230.

In both cases a program or a dedicated hardware, especially a hardware without a processor that performs instructions of a program, may analyze the movement of subject 408 and may be based on analyzing results issue at least one corresponding command to the optical output device.

To avoid detecting movements which are irrelevant to the quality of MR image in some of the procedure a calibration procedure may be used. Examples for irrelevant movements are: eye blinking, lip(s) movements, chin movement, yawning, nose wrinkling movement, eye brow movement, cheek movement, etc. The calibration may be done manually. However, an automatic or semi-automatic calibration procedure may comprise automatic face feature detection, see for instance:
- 3) Fernando De la Torre, Wen-Sheng Chu, Xuehan Xiong, Francisco Vicente, Xiaoyu Ding, Jeffrey Cohn, "IntraFace", IEEE Int. Conf. Autom. Face Gesture Recognition Workshops. 2015.

The recognition of eye regions and/or lip regions or areas, etc. may be followed by masking of the detected face features. Alternatively an MRI technician or an operator may manually tag irrelevant features among the detected features through a graphical user interface GUI, using for instance a touchscreen of control 130, 230 or a computer 260 connected to the disclosed system 100, 200. Alternatively an MRI technician or an operator may manually select the image area to be ignored while detecting moving features.

The method may further comprise a classification of the detected movement into two or more classes according to characteristics of the movement, including movement speed, displacement etc. In one example, two classes may be used:
- with one class defined as "movement within allowed range", and
- the second class defined as "movement out of the allowed range".

In a second example, three classes may be used:
- first class defined as "no movement",
- second class defined as "light movement", and
- third class defined as "excessive movement".

Alternatively only one, more than two or more than three classes may be used to distinguish various types and intensities of the movement.

The method may further comprise issuing a control signal to the output device 120. The control signal may dependent on the detected movement class. The control signal may preferably control optical output device 120, 220 to provide to the patient an information about the movement level. The information may be presented as various colors of for instance an LED (light emitting diode) light produced by optical output device 120, 220 e.g. green for class "no movement", yellow for class "light movement", red for class "excessive movement". However, the colors may be adjusted according to the number of classes and/or patient's requirements, e.g. to make colors distinguishable by the patient in case of e.g. color blindness or other physiological, anatomical or psychological conditions. Alternatively other methods of providing information through optical output device 120, 220 or another output device may be used, such as various frequency of LED blinking, changing intensity of generated light or fluent changes of LED color.

The disclosed method may comprise a task for the patient to minimize his/hers movements to keep the information provided by output device 120, 220 as close to the desired one as possible. Thus, the method may establish a movement based biofeedback method to minimize MRI artifacts. The method may gamify the MRI procedure for the patient with a strategy to reward the patient with positive information if he or she achieves low movement score during the procedure.

As mentioned in above, in one of the embodiments the image processing module or unit IPU may be integrated in a common housing with the control unit 130, 230. In control unit 130, 230, there may be an integrated LCD (liquid crystal device) video processor to digitize the analog signal from the camera. This processor may be responsible for controlling the control unit's screen, through its embedded TFT (thin film transistor) panel support. The signals dedicated for the TFT panel may be used simultaneously as input signals to the image processing module or unit IPU, where it can be processed using image processing algorithms. This is only one example. Various other scenarios may be considered. The main scenario may be to use the image processing module or unit IPU connected to the receiving unit 250, i.e. a unit that is outside of the MRI room 290.

Benefits and capabilities are:
- avoiding motion artefacts in MR images saves costs and time for patients and for clinics,
- more comfort and ease of the subject 408 during MR imaging,
- faster and more cost efficient movement detection compared to image detection that is based on MRI related medical image data, i.e. motion detection in real time, e.g. in less than 100 milliseconds or in less than 2 milliseconds,
- lesser involvement of the MRI technician in obligation to follow patient movement.

Other technical aspects may be:
- functional housing 304 and frame 400 design may meet medical standards, and/or
- lightweight and easy to install structure, especially housing 304 and/or frame 400, and/or
- possibility of convenient hanging of the camera device 110, 210 on the scanner's device 192 gantry, and/or
- a tripod or stand or frame 400 that allows one to adjust camera device 110, 210 position as desired, and/or
- possibility of directing the camera device 110, 210 at any part of the patient's body.

Although embodiments of the present disclosure and their advantages have been described in detail, it should be understood that various changes, substitutions and alterations can be made therein without departing from the spirit and scope of the disclosure as defined by the appended claims. For example, it will be readily understood by those skilled in the art that many of the features, functions, processes and methods described herein may be varied while remaining within the scope of the present disclosure. Moreover, the scope of the present application is not intended to be limited to the particular embodiments of the system, process, manufacture, method or steps described in the present disclosure. As one of ordinary skill in the art will readily appreciate from the disclosure of the present disclosure, systems, processes, manufacture, methods or steps presently existing or to be developed later that perform substantially the same function or achieve substantially the same result as the corresponding embodiments described herein may be utilized according to the present disclosure. Accordingly, the appended claims are intended to include within their scope such systems, processes, methods or steps. Further, it is possible to combine embodiments mentioned in the first part of the description with examples of the second part of the description which relates to Figures 1 to 6.

## Claims

1. System (100, 200) for giving feedback based on movement before or during medical imaging, comprising:
an optical camera device (110, 210) that is configured to generate image data of at least two optical images of a subject (408) or of a part of a subject (408) which can be arranged or which is arranged at a subject placing location (194) of a medical imaging device (192), and
a feedback signaling unit (120, 124) that is configured to generate based on movement data obtainable from the image data a feedback signal (Si1, Si2) that is perceptible by the subject (408) and/ or by an operator of a medical imaging device (192) and/or by a technician that is responsible for the medical imaging device.

2. System (100, 200) according to claim 1, comprising an image processing unit (130, 230, 250, 260, 600, IPU) that is coupled to the optical camera device (110, 210) and that is configured to process the least two images generated by the camera device (110, 210) in order to determine the movement of the subject (408) or the part of the subject (408) before or during medical imaging,
wherein the image processing unit (130, 230, 250, 260, 600, IPU) generates at least one output signal depending on the determined movement.

3. System (100, 200) according to claim 1,
wherein the feedback signal (Si1, Si2) changes if the orientation and/or location of the subject relative to the medical image device (192) changes by more than a predetermined amount, and/or
wherein the optical camera device (110, 210) is configured to operate within a static magnetic field of at least one tesla.

4. System (100, 200) according to claim 2 or 3, wherein the image processing unit (130, 230, 250, 260, 600, IPU) is configured to generate an intermediate score that indicates the movement during a time period that is preferably shorter than a time that is needed for the medical imaging,
wherein the score is preferably formed by summing up at least two values that indicate the amount of movement during the time period.

5. System (100, 200) according to one of the preceding claims, wherein the feedback signaling unit (120, 124) is integrated into the camera device.

6. System (100, 200) according to one of the claims 1 to 4, wherein the feedback signaling unit (120, 124) is a device that is separate from the camera device (110, 210), preferably the feedback signaling unit (120, 124) is integrated within a medical imaging device, e.g. a MRI machine (192).

7. System (100, 200) according to one of the preceding claims as far as referenced back to claim 2, wherein the image processing unit (120, 124, IPU) is comprised in the optical camera device (110, 210) or in a control unit (130, 230) for the optical camera device (110, 210) which is operatively coupleable to the control unit (130, 230).

8. System (100, 200) according to one of the claims 1 to 6 as far as referenced back to claim 2, wherein the image processing unit (250, 260, 600, IPU) is a device that is separated from the camera device (110, 210) and from a control unit (130, 230) of the camera device (110, 210).

9. System (100, 200) according to one of the preceding claims, wherein the image processing unit is configured to detect movements that are less than or equal to 3 mm, less than or equal to 2 mm or less than or equal to 1 mm,
wherein preferably the image processing unit is also configured to detect only movements that are more than and/or or equal with regard to a threshold value, preferably to 0.1 mm, more than or equal to 0.2 mm, more than or equal to 0.3 mm or more than or equal to 0.5 mm.

10. System (100, 200) according to one of the preceding claims as far as referenced back to claim 2, wherein the image processing unit (130, 230, 250, 260, 600, IPU) is configured to determine movements within a time interval of less than half a second or less than 100 milliseconds between commencement of capturing the last image that is considered for determining the current movement and the generation of the output signal.

11. System (100, 200) according to one of the preceding claims as far as referenced back to claim 2, wherein the image processing unit (130, 230, 250, 260, 600, IPU) comprises a masking unit or a masking functionality that is configured to identify and/or mask (518) at least one area or other feature of the subject, preferably areas of the eyes and/or of the lips of the subject (408),
wherein the image processing unit (130, 230, 250, 260, 600, IPU) is configured to disregard movement inside of the at least one area.

12. System (100, 200) according to claim 11, wherein the masking unit or a masking functionality is configured to determine the areas or features automatically or semi automatically.

13. System (100, 200) according to one of the preceding claims as far as referenced back to claim 2, wherein a first trigger signal is issued by the image processing unit (130, 230, 250, 260, 600, IPU) for a medical imaging system to repeat only a part of a current sequence, preferably the past k-space line in MRT, if movement of the subject (409) exceeded a limit and the patient got back to his/her position, and/or wherein a second trigger signal is issued by the image processing unit (130, 230, 250, 260, 600) for the medical imaging system if movement exceeded the limit and the subject (409) did not return to his/her previous position, wherein preferably the second trigger signal triggers the MRI system (102) to repeat the whole sequence.

14. Image processing unit (130, 230, 250, 260, 600, IPU), preferably an image processing unit (130, 230, 250, 260, 600, IPU) of a system (100, 200) according to one of the preceding claims, comprising:
an input unit (In), an output unit (Out) and a processing unit (Pr),
wherein the input unit (In) is configured to receive image data of at least two images from an optical camera device (110, 210),
wherein the processing unit (Pr) is configured to process at least two optical images in order to determine the movement of a subject (408) or a part of the subject (408) before or during a medical imaging that is preferably non-optical,
wherein the processing unit (Pr) is configured to generate at least one output signal or output data depending on the determined movement,
and wherein the output unit (Out) is configured to send the output data or the output signal to a feedback signaling unit (120, 124).

15. Method (500) of giving feedback based on motion before or during medical imaging, comprising:
generating (520, 532) movement data of a subject (408) or of a part of the subject (408) from which at least one medical image is generated during and/or before generating the at least one medical image, and
sending(526) to the subject (408) a feedback signal (Si1, Si2) that depends on the amount of the determined movement.

16. Method (500) according to claim 15, comprising:
- determining (522) movement of the subject (408) or of the part of the subject (408) based on at least two images that are generated optically.

17. Method (500) according to claim 15 or 16, comprising
- generating (529) non-optically at least one medical image of the inside of the subject (408).

18. Method (500) according to any one of the claims 15 to 17, wherein a feedback signal (Si1, Si2) is presented using a computer game wherein preferably an object or a character adjusts its movement and/or its behavior according to the movement of the subject (408).

19. Method (500) according to any one of the claims 15 to 18, wherein the medical image is an MR image of an MRI machine (192).

20. Method (500) according to any one of claim 15 to 19, wherein at least two or at least three movement levels are defined and wherein the determined movement is classified according to the specified movement levels, and
wherein preferably different feedback signal (Si1, Si2) are generated depending on the classification.

21. Method (500) according to any one of the claims 15 to 20, wherein the subject (408) is instructed to control and/or to minimize his/her movement to achieve predefined feedback, preferably on a feedback signaling unit (120, 124, 220) and/or preferably constantly over a defined time of a medical imaging procedure.

22. Method (500) according to any one of the claims 15 to 21, comprising:
matching (522) at least one keypoint within the at least two optical images and
determining a value that represents the movement of the at least one keypoint,
comparing (524) the value with at least one threshold value (TH),
giving (526) to the subject (408) a feedback signal that indicates that the value is above the at least one threshold value (TH) and/or feeding back (528) to the subject (408) a signal that indicates that the value is below and/or equal to the at least one threshold value (TH).

23. Computer program product comprising computer readable program code with instructions which, when loaded and/or executed on a processor (Pr), cause the processor (Pr) to carry out at least one of, an arbitrarily selected plurality of, or all of the method steps according to any one of the claims 15 to 22.

24. System (100, 200), comprising:
an optical camera device (110, 210) that is configured to generate image data of at least two optical images of a subject (408) or of a part of a subject (408) which can be arranged at a subject placing location (194) of a medical imaging device (192), and
at least one trigger unit of:
- a first trigger unit that is configured to generate based on the image data a first trigger signal for an medical imaging system to repeat only a part of a current sequence of images if movement of the subject (409) exceeded a limit and the subject (408) got back to his/her position, and/or
- a second trigger unit that is configured to generate a second trigger signal based on the image data for the medical imaging system if movement exceeded the limit and the subject (408) did not return to his/her previous position, wherein preferably the second trigger signal triggers the medical imaging system to repeat the whole sequence of images.
